(19) Europäisches Patentamt

European Patent Office

Office européen des brevets

(11) Numéro de publication: **0 210 939**
**A1**

(12) **DEMANDE DE BREVET EUROPEEN**

(21) Numéro de dépôt: 86450012.9

(22) Date de dépôt: 11.06.86

(51) Int. Cl.⁴: **A 61 L 2/04**
**A 61 L 2/02**

(30) Priorité: 12.06.85 FR 8508995

(43) Date de publication de la demande:
04.02.87 Bulletin 87/6

(84) Etats contractants désignés:
AT BE CH DE FR GB IT LI LU NL SE

(71) Demandeur: Ituarte, Angel
3 rue Lyautey
F-31600 Muret(FR)

(72) Inventeur: Ituarte, Angel
3 rue Lyautey
F-31600 Muret(FR)

(74) Mandataire: Ravina, Bernard
Cabinet Bernard RAVINA 24, boulevard Riquet
F-31000 Toulouse(FR)

(54) Procédé et appareil pour nettoyer et stériliser les lentilles de contact.

(57) La présente invention concerne un procédé et un appareil pour nettoyer les lentilles de contact par l'association d'un traitement thermique avec un traitement mécanique vibratoire.

Le traitement thermique est constitué:

- d'une première montée en température du liquide contenant les dites lentilles depuis la température ambiante (T1) à une température (T2) à laquelle la matière collée aux lentilles ne subit pas de dénaturation,

- d'une série d'arrêts et de mises en marche d'échauffement permettant l'oscillation en température entre la température T2 et une autre température T3 inférature entre la température T2 et une autre température T3 inférieure à T2 et supérieure à T1,

- et enfin d'une deuxième montée en température depuis T3 à une autre température T4 supérieure à T2.

Le traitement vibratoire est à appliquer d'une façon indépendante par rapport au traitement thermique, soit pendant toute la période entre T1 et T2 soit pendant celle entre T1 et T4.

Fig 2

EP 0 210 939 A1

La présente invention est relative à un procédé et à un appareil pour nettoyer et stériliser les lentilles de contact.

On entend par le nettoyage de faire décoller la matière étrangère collée sur les lentilles.

Une partie de cette matière est constituée par des protéines des larmes qui coagulent sous l'effet du traitement thermique à partir d'un certain seuil de température. Par contre une température supérieure à ce seuil est nécessaire pour avoir une stérilisation plus complète, d'où la nécessité d'effectuer le nettoyage à une température inférieure au dit seuil ou de l'effectuer avant le traitement thermique pour éviter la coagulation des protéines qui sont beaucoup plus difficile à décoller une fois qu'elles sont dénaturées.

Enfin, la température maximale du traitement thermique ne doit pas altérer les lentilles elles-mêmes et notamment les lentilles souples.

Plusieurs appareils offrent des cycles de traitement thermique, certains proposent plusieurs cycles au choix (voir par exemple FR 2454 811).

Les cycles de traitement thermique sont constitués en général d'une montée en température, d'une maintenance de cette température pendant une durée prédéterminée et enfin d'une descente de la température, c'est-à-dire d'une coupure de chauffage.

On connaît plusieurs appareils qui associent le nettoyage par l'application de vibrations mécaniques, généralement ultrasoniques, et le traitement thermique.

Le principe du cycle d'un traitement thermique est souvent équivalent à celui qu'on vient de décrire.

La présente invention propose un autre cycle de traitement thermique plus efficace associé à un moyen de nettoyage par vibration mécanique.

Le dit cycle expose les lentilles à stériliser à des variations thermiques plus élaborées que le cycle traditionnel, en vue d'accentuer l'efficacité du dit traitement ceci, d'une façon indépendante de l'application des vibrations mécaniques.

A cet effet, et suivant une disposition, la présente invention est relative à un procédé pour nettoyer et stériliser les lentilles de contact maintenues dans un milieu liquide, par l'association d'un traitement thermique, essentiellement pour les stériliser, avec un traitement mécanique vibratoire essentiellement pour décoller la matière qui y est collée, ces deux traitements étant appliqués simultanément pendant toute la période de traitement ou pendant une partie de celle-ci, caractérisé en ce que le traitement est constitué :

- d'une première montée en température du dit liquide, de la température ambiante (T1) à une température (T2) à laquelle la matière collée aux lentilles ne subit pas de dénaturation,
- d'une série d'arrêts et de mises en marche d'échauffement espacés dans le temps de telle façon que la température du dit liquide oscille entre T2 et une autre température T3 inférieure à T2 et supérieure à T1,
- et enfin, d'une deuxième montée en température depuis T3 à une température T4 supérieure à T2 et à laquelle la stérilisation complète est effectuée, en ce que le traitement mécanique vibratoire est appliqué d'une façon indépendante par rapport à l'application thermique dans toute la période entre T1 et T2

soit dans celle entre T1 et T4.

La partie du traitement thermique ci-avant décrit et constituée par une série de chauffages répétés en alternance avec des périodes de refroidissement, est connu sous le nom stérilisation de TRYNDAL.

Suivant une autre disposition, la présente invention est relative à un appareil pour nettoyer et stériliser les lentilles de contact du type comprenant un transducteur piézoélectrique pour produire les vibrations mécaniques, celui-ci étant mis en contact avec le fond d'un boîtier à remplir d'un liquide convenable et présentant un logement adapté pour recevoir un étui pour les lentilles de contact, un circuit électronique comportant une résistance thermosensible (R2), une unité de chauffage et un circuit d'alimentation caractérisés par

- un transducteur piézoélectrique et un circuit de commande alimenté tous les deux par une même alimentation,
- une unité de chauffage alimentée directement par le secteur et géré,de même que le transducteur piézoélectrique,par le dit circuit de commande.

La présente invention sera mieux comprise à la lecture de la description détaillée ci-dessous, illustrée par des dessins dans lesquels :

- la fig. 1 est un schéma électronique qui présente le circuit d'alimentation et les différents composants qui commandent l'unité de chauffage R et le transducteur piézoélectrique P.
- la fig. 2 est une courbe qui présente l'évolution en température dans le boîtier (B) qui contient l'étui des lentilles.

- la fig. 3 est un schéma qui montre l'emplacement du transducteur piézoélectrique (P), de l'unité de chauffage R et de la résistance thermosensible R2 par rapport au boîtier (B).
- les fig. (4a) et (4b) montrent respectivement une partie de la courbe de l'évolution en température dans le boîtier (B), présentée dans la fig. 2, et la partie correspondante de la courbe d'évolution en température de l'emplacement de la résistance thermosensible (R2).


Le procédé, selon l'invention, consiste à associer un traitement thermique spécifique avec un traitement mécanique vibratoire, en vue de stériliser et nettoyer les lentilles de contact.


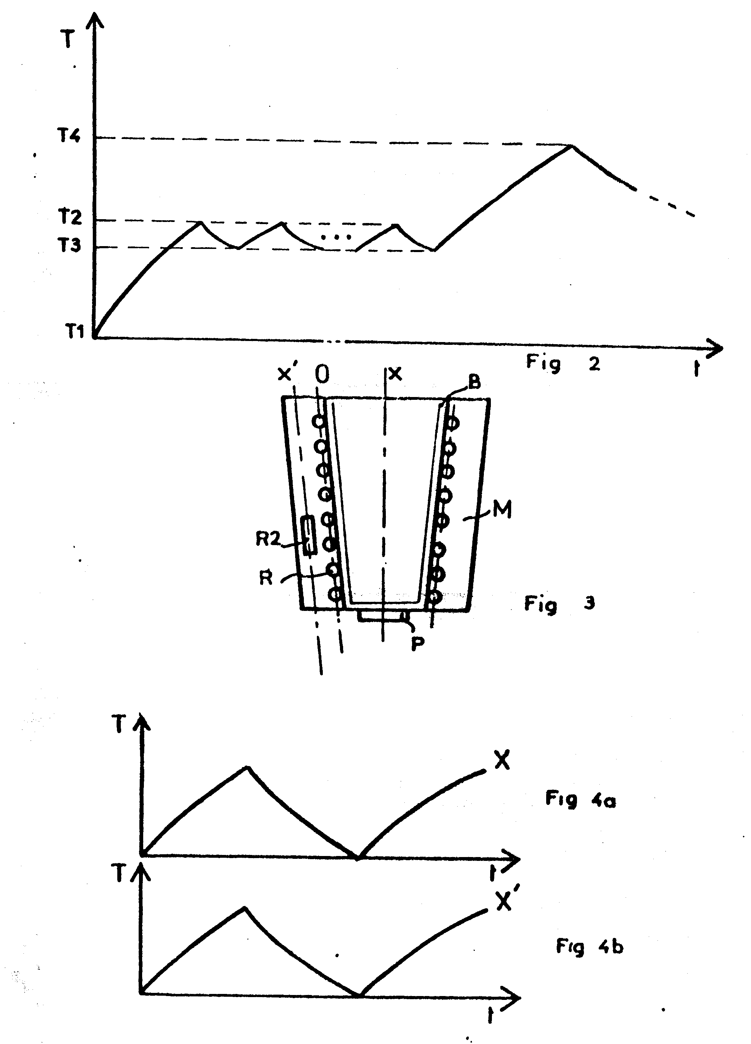

Le dit traitement thermique consiste à chauffer le milieu liquide, dans lequel les lentilles de contact sont maintenues, depuis la température ambiante (T1) jusqu'à une température (T2) à laquelle la matière collée aux dites lentilles ne subit pas de dénaturation, c'est-à-dire de coagulation (fig. 2). Nos essais ont montré que cette température doit être inférieure à 60° C.


L'énergie thermique fournie au liquide contenant les lentilles n'est pas suffisante pour faire démarrer la coagulation des proéines, provenant des larmes des yeux, collées aux lentilles, mais agite le liquide qui les contient ainsi que les molécules de protéines elles-mêmes. Ceci favorise l'action de vibrations mécaniques qui sont appliquées au dit liquide en même temps pendant cette phase de traitement thermique.


Une fois que la température du liquide atteint la température T2, on coupe le chauffage et on laisse le liquide refroidir jusqu'à une température T3 supérieure à T1, on chauffe de nouveau jusqu'à T2 puis on coupe de nouveau le chauffage. On répète cette manoeuvre pour que la température du liquide oscille entre T2 et T3 un nombre de fois désiré.

L'énergie à dissiper dans le liquide par l'application des vibrations mécaniques ne doit pas émpêcher le refroidissement du dit liquide. Ceci est possible en choisissant convenablement la puissance du transducteur piézoélectrique.

On chauffe le liquide jusqu'à une température T4 supérieure à T2 et à laquelle une stérilisation complète est effectuée.
Le fait de faire osciller la température entre T2 et T3 aide à rendre la stérilisation efficace dans cette gamme de température, c'est-à-dire qu'il aide à rompre le phénomène d'adaptation manifesté par les microorganismes vis à vis du changement thermique dans un seul sens.

Il est bien évident que ce fait de faire osciller la température peut être effectué à n'importe quel niveau de température, à un seul ou à plusieurs niveaux.

Le traitement vibratoire mécanique peut être appliqué au dit liquide soit pendant toute la période entre T1 et T2 soit pendant celle entre T1 et T4. Dans ce dernier cas, le nettoyage c'est-à-dire le décrochement de la matière collée aux dites lentilles aura lieu pendant toute la durée du traitement thermique : d'abord pour décoller les protéines à basse température et puis pour décrocher d'éventuelles autres matières qui peuvent y être accrochées et qui ne subissent pas de dénaturation sous l'effet thermique.

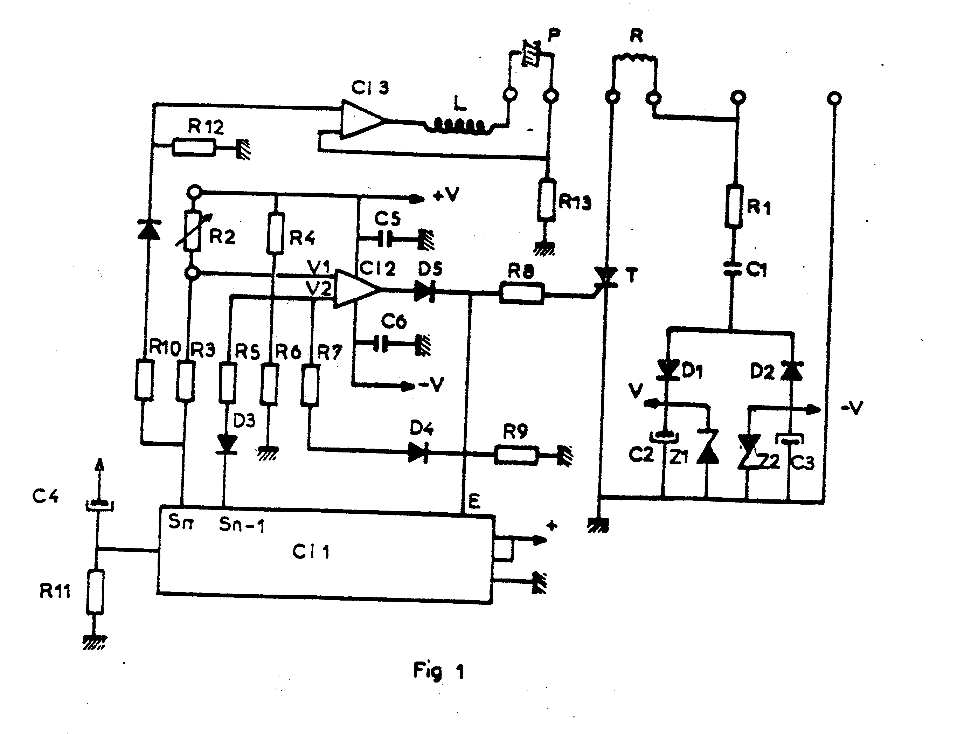

L'appareil pour la mise en oeuvre du procédé, et dont le schéma électronique est présenté sur la figure 1, est donné à titre d'exemple indicatif.

Cet appareil comporte une unité d'alimentation qui donne une fois branchées au secteur, deux tensions opposées de +V et de -V. Elle est constituée d'une résistance R1 et d'un condensateur C1 reliés en série, de deux ensembles chacun étant constitué d'une diode, d'un condensateur et d'une diode zener, cette dernière est destinée à limiter la tension. Ces deux ensembles fonctionnent en sens opposés.

Le premier ensemble est constitué par la diode D1, le condensateur C2 et la diode zener Z1. Le deuxième ensemble est constitué par la diode D2, lecondensateur C3 et la diode zener Z2.

La tension V est divisée entre deux résistances, une résistance variable thermosensible R2 et une résistance fixe R3 pour donner une première tension V1 sur leur ligne de liaison. La même tension V est divisée entre d'un côté une résistance R4 et d'autre côté une série de résistances R5, R6 et R6, pour donner une deuxième tension V2 sur la ligne de liaison de la résitance R4 et la dite série.

Ces deux tensions sont appliquées sur les deux entrées, non-inverseuse et inverseuse, nommées respectivement l'entrée (+) et l'entrée (-) d'un comparateur de tension (CT2), par exemple un amplificateur opérationnel, dont la sortie change la tension en fonction de la différence entre les deux tensions appliquées aux entrées. La tension sur la dite sortie prend soit une valeur de signe positive soit la même valeur mais de signe négatif. La dite valeur est sensiblement égale à V.

La tension de sortie de CI2 est appliquée à la gachette d'un thyristor (Th) pour le rendre passant ou non, en vue d'autoriser l'alimentation d'une unité de chauffage (R) alimentée directement par le courant du secteur. Si cette tension de sortie est positive c'est-à-dire si V1 est inférieure à V2, la résistance R se chauffe quittant la température ambiante T1 à la suite de l'autorisation du passage de courant à travers (Th). La résistance R7 est bloquée à cause du signal positif de la sortie opposé au signal V2 de l'entrée (+) par l'intermédiaire d'une diode (D4) passante dans la direction entrée (+)-sortie du CI2. La résistance thermosensible située à proximité de (R) se chauffe aussi et change de valeur. Dans le montage présenté dans la fig. 1 la (R2) a un coefficient de température négatif, donc la tension V1 augmente. Lorsque V1 devient supérieure à V2, c'est-à-dire lorsque la température T2 est atteinte, la tension à la sortie devient négative, par conséquent le thyristor coupe l'alimentation de (R) et la résistance (R7) devient passante, ce qui fait baisser la valeur de V2. Ceci permet le refroidissement de l'ensemble constitué par un boîtier (B) dans lequel les lentilles sont maintenues dans un liquide adequat, l'unité de chauffage et la résistance thermosensible (R2).

Pour que le chauffage recommence de nouveau la tension V1 doit être inférieure à la nouvelle tension V2 c'est à dire que la température de l'ensemble doit être inférieure à T2. Une fois que la tension V1 devient inférieure à la nouvelle tension V2, à cause du refroidissement, le chauffage recommence de nouveau à partir de la température T3 à laquelle V1 = V2 jusqu'à T2.

Cette mise sous ou hors tension de l'unité de chauffage se répète un nombre désiré de fois faisant osciller la température entre T2 et T3.

L'écart entre ces deux températures est calculable en faisant varier la valeur de la résistance R7 par rapport aux R5 et R6.

La diode D5 empêche le passage du signal négatif qui apparaît à la sortie du CI2 lorsque V1 devient supérieur à V2.

Il est évident qu'on peut utiliser une résistance thermosensible de coefficient de température positif à la place de celle qui est utilisée dans le circuit présenté, tout simplement en interchangeant sa place avec celle de la résistance R3.

Un compteur (CI1) a une entrée (E) et n sortie (S) : S1, S2 ... Sn-1, Sn ; ce compteur présent un signal dont la hauteur est sensiblement égale à V à ses sorties l'une après l'autre dans l'ordre de 1 à n. Toutes les sorties, sauf celle qui présent le dit signal, sont reliées à la masse.

L'entrée E du CI1 est reliée à la sortie du CI2. Chaque fois qu'un signal positif paraît à la dite sortie c'est-à-dire chaque fois que la valeur de V2 dépasse la valeur de V1, le compteur CI1 est incrémenté d'un pas. Une de ces sorties, par exemple la sortie Sn-1, est reliée à la résistance R5 par l'intermédiaire d'une diode D3 passante dans le sens entrée (+) du CI2 - sortie Sn-1.

0210939

Une fois que le signal du compteur CI1 est présenté sur la sortie Sn-1, la résistance R5 n'est plus traversée par le courant et la mise sous tension de l'unité de chauffage R continue jusqu'à une température T4 supérieure à T2. Il est bien évident que cette température est calculable par le choix de la résistance R5 relativement aux R6 et R7. Une fois que cette température (T4) est atteinte l'unité de chauffage est mise hors tension et un signal positif apparaît sur la sortie suivante du compteur CI1.

Le transducteur piézoélectrique (P) est alimenté, par la même alimentation que le circuit de commande, en courant alternatif pour donner des vibrations mécaniques à appliquer au liquide contenant les dites lentilles de contact.

Le dit transducteur piézoélectrique (P), possédant certain effet capacitif, est monté dans un circuit résonant (LC) en coopération avec une self inductance (L). La maintenance de cette résonance dans le temps est assurée par un moyen de comparaison de tension, par exemple un amplificateur opérationnel (CI3) identique à (CI2) et dont la sortie change la valeur soit positivement soit négativement selon la différence des tensions appliquées à ses deux entrées (+) et (-).

En alimentant le CI3, il apparaît à sa sortie un signal provisoire quelconque soit positif soit négatif dû à sa construction électronique. Ce signal provoque, après avoir traversé (L) et (P), l'apparition d'une tension de sens opposé sur l'entrée (+) du CI3, ce qui mène à compléter le chargememnt du (P), comme condensateur, jusqu'au niveau maximal de la sortie du (CI3).

A l'arrêt du chargement du (P), l'entrée (+) du CI3 change de tension par rapport à la masse à cause de l'arrêt du passage de charge dans la résistance R13. Ce demi-cycle qu'on vient de décrire se répète dans un sens inverse à la suite du changement de la tension sur la sortie du CI3. C'est ainsi qu'un signal alternatif carré est appliqué sur le transducteur (P).

L'avantage de monter le transducteur piézoélectrique (P), comme condensateur, en un circuit de résonance réside en ce que la tension effective appliquée sur lui est beaucoup plus grande que la tension d'alimentation. Le rapport entre la tension effective et la tension d'alimentation est proportionnel d'une part inversement à la résistance ohmique du circuit et d'autre part directement à la racine carrée de l'inductance divisée par la racine carrée de la capacité.

A l'apparition du signal positif à la sortie Sn du (CI1), ce signal se retrouve sur l'entrée (-) du (CI2) et sur l'entrée (-) du (CI3). La tension sur ces deux entrées sera plus élevée que celle appliquée sur l'entrée (+) de chaque amplificateur ,ce qui rend la tension à leur sortie négative en permanence et par conséquent met le chauffage hors tension et bloque l'oscillation du circuit (RC).

Si la sortie (Sn) est la dernière parmi les sorties du (CI1), la coupure du chauffage et le blocage du circuit de résonance seront maintenus jusqu'à une nouvelle mise à zéro du CI1.

Il est évident que le fonctionnement de la résistance (R5) peut être répété par l'ajout d'une ou plusieurs résistance reliées avec elle en parallèle et connectées chacune à une des sorties du CI1 pour faire varier à tour de rôle la tension V2 appliquée sur l'entrée (+) du CI2.

L'avantage d'utiliser un transducteur piézoélectrique d'une telle puissance à pouvoir l'alimenter par la même alimentation que les composants du circuit de commande c'est-à-dire (CT1), (CT2) et T4, nous permet de n'utiliser ni de transformateur ni de composants de puissance. Ceci rend l'appareil plus léger et plus compact et nous évite de prévoir des systèmes d'aération dans le cas d'utilisation de composants de puissance.

L'unité de chauffage (R) est noyée dans une masse M d'une matière appropriée, classiquement connue, d'une forme qui épouse le boîtier (B), pour transférer la chaleur, d'un côté au dit boîtier dans lequel les lentilles de contact sont placées dans un liquide adequat, et d'un autre côté à la résistance thermosensible qui est soit, noyée elle aussi dans la masse (M), soit collée contre elle de l'extérieur, soit mise à distance d'elle. Le positionnement de l'unité de chauffage (R) et celui de la résistance thermosensible (R2) sont effectués de telle façon que la variation en température, à la suite de la mise sous ou hors tension de l'unité de chauffage, sur l'axe X du boîtier (B), soit identique à celle de la surface X' à laquelle la (R2) est placée (fig. 4a et fig. 4b).

Ces positionnements sont calculés en prenant en considération la capacité et la conductivité thermiques de la matière du boîtier (B), du liquide qui le remplit et de la masse (M).

Le fonctionnement de l'appareil est le suivant :

- à la suite de la mise sous tension :

a) la tension V1 est inférieure à V2, le thyristor (Th) est passant et par conséquent l'unité de chauffage (R) est mise sous tension et la résistance (R7) est bloquée.

b) le transducteur piézoélectrique est alimenté en courant alternatif et envoie des vibrations mécaniques au liquide qui contient les lentilles de contact.

- Lorsque V1 devient supérieure à V2, la température T2 est atteinte, le thyristor (Th) devient non-passant et la tension V2 diminue à la suite de déblocage de la résistance R7, permettant le refroidissement de l'ensemble.

- Le chauffage se met en marche de nouveau lorsque V1 devient inférieur à la nouvelle valeur de V2.

- Cette dernière étape se répète un nombre désiré de fois faisant osciller la température entre T2 et T3.

- A la suite de l'apparition d'un signal sur la sortie Sn-1 du (CI1), la résistance (R5) devient bloquée ce qui fait augmenter la tension V2 et permet de monter la température jusqu'à la température T4, il est à noter que la résistance R7 sera bloquée elle aussi.

- A la suite de l'apparition d'un signal sur la sortie Sn du

**0210939**

(CI1), la tension V1 sur l'entrée (-) du (CI3) prennent une valeur supérieure, dans tous les cas, à celles sur les entrées (+) de ces deux comparateurs, ce qui met hors tension le chauffage et empêche l'oscillation du circuit (RC).

Il est à noter que le boîtier (B) peut être muni d'un agitateur magnétique dont le circuit d'inductance est alimenté par la même alimentation que le transducteur piézoélectrique (P) et le circuit de commande. Ceci pour disperser les particules détachées des lentilles de contact dans la totalité de liquide.

Il va de soi que la présente invention peut prendre toutes modifications et toutes variantes de la présente demande.

REVENDICATIONS

1. Procédé pour nettoyer et stériliser les lentilles de contact maintenues dans un milieu liquide par l'association d'un traitement thermique, essentiellement pour les stériliser, avec un traitement mécanique vibratoire, essentiellement pour décoller la matière qui y est collée, ces deux traitements étant appliqués simultanément pendant toute la période de traitement ou pendant une partie de celle-ci, caractérisé en ce que le traitement thermique est constitué :

- d'une première montée en température du dit liquide de la température ambiante (T1) à une température (T2) à laquelle la matière collée aux lentilles ne subit pas de dénaturation.

- d'une série d'arrêts et de mises en marche d'échauffement espacés dans le temps de telle façon que la température du dit liquide oscille entre T2 et une autre température T3 inférieure à T2 et supérieure à T1.

- et enfin, d'une deuxième montée en température depuis T3 à une température T4 supérieure à T2 et à laquelle la stérilisation complète est effectuée, en ce que le traitement mécanique vibratoire est appliquée soit entre T1 et T2 soit entre T1 et T4.


2. Appareil pour nettoyer et stériliser les lentilles de contact du type comprenant un transducteur piézoélectrique pour produire des vibrations mécaniques, celui-ci étant mis en contact avec le fond d'un boîtier à remplir d'un liquide convenable et présentant un logement adapté pour recevoir un étui pour les lentilles de contact, un circuit électronique comportant une résistance thermosensible (R2), une unité de chauffage et un circuit d'alimentation caractérisé par un moyen de comparaison de tensions ,dit comparateur, disposant de deux entrées et dont la sortie commande la mise de sous ou hors tension de l'unité de chauffage, sur ces deux entrées, on applique d'un côté une première tension variable (V1) relative à la résistance thermosensible (R2) et d'un autre coé une deuxième tension variable programmable (V2), ce qui conduit à changer la valeur de la sortie du dit comparateur et par la suite la mise sous ou hors tension de la dite unité de chauffage.

3. Appareil selon la revendication R2 caractérisé en ce que la première tension (V1) résulte de la division d'une tension d'une valeur constante V entre la résistance thermosensible (R2) et une autre résistance constante (R3) et en ce que la deuxième tension (V2) est le résultat de la division de la même tension de la même valeur constante (V) entre d'un côté une résistance(R4) et d'un autre côté une série de résistances (R5),(R6) et(R7), certaines d'entre elles étant autorisées ou non à faire passer le courant c'est-à-dire à participer ou non à déterminer la valeur équivalente de la dite série de résistances.

4. Appareil selon la R3 caractérisée en ce que les résistances R5 et R7 sont branchées, l'une à la sortie de CI2 et l'autre à une des sorties d'un compteur CI1, branchement fait par l'intermédiaire de deux diodes D3 et D4 respectivement de telle façon qu'elles soient passantes seulement sous l'effet de poussée de la tension V2.

5. Appareil selon la R2 dans lequel le transducteur piézoélectrique (P) est alimenté en courant alternatif caractérisé en ce que le transducteur piézoélectrique (P) est monté en circuit de résonance LC dans lequel l'effet capacitif est dû au transducteur et l'alimentation est attirée par la sortie d'un moyen de comparaison de tension CI3 identique à CI2 dont une des deux entrées est reliée normalement à la masse.

6. Appareil selon la R2, R4 et R5 dans lequel un compteur (IC1) dont les sorties présentent l'une après l'autre, un signal dont la hauteur est sensiblement égale à V, ces sorties étant reliées à la masse sauf celle qui présente le signal, est incrémenté d'un pas chaque fois qu'un signal convenable est appliqué à son entrée, caractérisé en ce que la dite entrée du (CI1) est reliée à la sortie du (CI2) par l'intermédiaire d'une diode(D5), passante dans le sens sortie (CI2)-entrée du (CI1), en ce qu'une sortie du (CI1) est reliée à l'entrée normalement reliée à la masse du (CI3).

Fig 1

Fig 2

Fig 3

Fig 4a

Fig 4b

Office européen
des brevets

**RAPPORT DE RECHERCHE EUROPEENNE**

EP    86 45 0012

## DOCUMENTS CONSIDERES COMME PERTINENTS

| Catégorie | Citation du document avec indication. en cas de besoin. des parties pertinentes | Revendication concernée | CLASSEMENT DE LA DEMANDE (Int Cl 4) |
|---|---|---|---|
| Y | FR-A-2 240 742  (R. McLURE)<br>*    Page    6,    lignes    18-40;<br>revendications 1,3,19,22-25,43 *<br><br>--- | 1-6 | A 61 L    2/04<br>A 61 L    2/02 |
| Y | EP-A-0 126 665  (J.E. PRAT)<br>* Page 8, lignes 18-27;  page  9,<br>lignes 14-18 *<br><br>--- | 1 | |
| Y | EP-A-0 078 614  (AMERICAN<br>STERILIZER)<br>* Pages 7,8; revendication 1 *<br><br>--- | 1 | |
| A | EP-A-0 031 152  (LABORGERÄTE &<br>MEDIZINTECHNIK WEBER)<br>* Revendications 1,3; figure 5 *<br><br>--- | 1 | |
| Y | US-A-3 973 760  (I. BROWNING et<br>al.)<br>*  Revendication  1;  colonne  5,<br>lignes 57-68; figure 4 *<br><br>----- | 2-6 | **DOMAINES TECHNIQUES RECHERCHES (Int Cl 4)**<br><br>A 61 L |

Le present rapport de recherche a été établi pour toutes les revendications

| Lieu de la recherche<br>**LA HAYE** | Date d'achèvement de la recherche<br>**07-10-1986** | Examinateur<br>**PELTRE CHR.** |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille. document correspondant

OEB Form 1503 03 82